# EUROPÄISCHE PATENTSCHRIFT

(11) **EP 3 548 127 B1**
(45) Veröffentlichungstag und Bekanntmachung des Hinweises auf die Patenterteilung: **13.09.2023**
(21) Anmeldenummer: 17838032.5
(22) Anmeldetag: 20.12.2017
(51) Int. Cl.: B05B 17/06, A61M 15/00, B05B 17/00

(54) **VERNEBLEREINHEIT MIT DIREKT ANSCHLIESSBARER AMPULLE**
ATOMIZING UNIT WITH DIRECTLY CONNECTABLE AMPOULE
UNITÉ DE NÉBULISATION MUNIE D'UNE AMPOULE POUVANT ÊTRE RACCORDÉE DIRECTEMENT

(30) Priorität: 02.01.2017 DE 102017100012
(43) Veröffentlichungstag der Anmeldung: 09.10.2019
(73) Patentinhaber: Kern, Stefan, 63820 Elsenfeld (DE)
(72) Erfinder: Kern, Stefan, 63820 Elsenfeld (DE)
(74) Vertreter: Pöhner, Wilfried Anton
(86) Internationale Anmeldenummer: PCT/DE2017/101098
(87) Internationale Veröffentlichungsnummer: WO 2018/121813

(56) Entgegenhaltungen:
- EP-A1- 2 987 520
- EP-A2- 2 062 608
- WO-A1-96/28205
- DE-A1-102005 038 619
- DE-B3-102008 054 431
- US-A- 5 819 730
- US-A- 6 145 703
- US-A1- 2003 140 921

## Beschreibung

Vorliegende Erfindung befasst sich mit einer Verneblereinheit mit Ampulle gemäß der Oberbegriffe der unabhängigen Ansprüche.

Um Atemwegs- und Lungenerkrankungen zu behandeln muss ein wirksames Medikament möglichst vollständig bis an den Ort der Erkrankung gebracht werden, das heißt bis in die Bronchien, Alveolargänge oder in die Lungenbläschen. Hierzu wird üblicher Weise ein Medikament als Lösung vorbereitet, und mittels eines Verneblers fein zerstäubt bevor es durch den Patienten inhaliert wird.

Übliche aus dem Stand der Technik bekannte Vernebler, welche auch als mobile Geräte erhältlich sind, bestehen aus vier wesentlichen Komponenten:
Das Herz des Verneblers ist die sogenannte Verneblereinheit, welche eine mikroperforierte Membran enthält, die mittels eines Schwingungserzeugers, üblicherweise ein Piezokristall, in schnelle Schwingungen versetzt wird, üblicherweise im Ultraschallbereich, wodurch die direkt an die Membran anliegende Medikamentenlösung in die mikrometergroßen Löcher der Membran hineingedrückt und auf der Ausgangsseite der Membran als ein Nebel von feinen Tröpfchen abgegeben wird. Üblicherweise ist die Membran umfänglich mit dem kreisringförmigen Schwingungserzeuger direkt ohne, oder indirekt mit einer Trägerplatte, mechanisch zu einer zusammenhängenden Einheit, dem Aerosolerzeuger, gekoppelt. Auf die Verneblereinheit wird entweder ein Schlauch mit Mundstück oder direkt ein Mundstück aufgesetzt. Des Weiteren gehört zu einem Vernebler eine Steuereinheit, welche die zur Ansteuerung des Schwingungserzeugers nötigen Signale erzeugt. Auf der Zuführungsseite der Membran, in die Verneblereinheit integriert befindet sich üblicherweise ein Reservoir für die Medikamentenlösung, welches einen ausreichend dimensionierten Hohlraum bereitstellt, welcher üblicherweise mittels einer Kappe fest verschließbar ist, damit im Reservoir vorhandene Flüssigkeit auch beim Transport des Verneblers in gefülltem Zustand nicht verloren geht.

Eine effektive und nachvollziehbare Inhalationstherapie stellt verschieden Anforderungen an einen Vernebler, besonders hinsichtlich Hygiene und Dosierung.

Eine genau Dosierungsüberwachung ist wichtig, damit sichergestellt ist, dass bei den üblicherweise mittels Inhalation verabreichten hochwirksamen Medikamenten der Patient auch wirklich eine als optimal bekannte Dosis inhaliert. Dabei ist ein Problem, dass bei üblichen Verneblern der Anwender eine zu zerstäubende bzw. zu inhalierende Flüssigkeitsmenge selbst aus einem größeren Medikamentenbehälter abmessen und in das Reservoir füllen muss. Dabei kann es leicht zu Ungenauigkeiten kommen. Auch wenn die Flüssigkeit schon in Inhalationsgerechten Mengen abgemessen vorliegt kann beim Umfüllen vom Medikamentenbehälter in das Reservoir ein Teil verlorengehen.

Um dies zu vermeiden, wurde vorgeschlagen die Medikamentenbehälter als Ampullen auszubilden, welche direkt in eine Ampullenaufnahme der Verneblereinheit eingesetzt werden können. Beispielsweise offenbart das Dokument WO 02/074373 A1 ein Flüssigkeitszuführungssystem und einen damit ausgestatteten Vernebler, aus einem Gehäuse mit einer Ampullenaufnahme und einem Flüssigkeitsannahmebereich. Die dazugehörige Ampulle verfügt an ihrem oberen wie unteren Ende über je ein Plättchen, welches beim Entfernen eine wohldefinierte Öffnung in der Ampulle hinterlässt. Die Ampulle wird vor dem Einsetzen zunächst am unteren Ende durch Abbrechen des dortigen Plättchens geöffnet, dann eingesetzt und erst dann zur Schaffung einer Ventilationsöffnung auch das obere Plättchen entfernt.

Ein Nachteil dieses Systems ist, dass bei unsachgemäßer Handhabung durch das Öffnen außerhalb der Ampullenaufnahme eine Kontaminierung der Medikamentenflüssigkeit durch Schmutz oder Keime des Anwenders möglich ist. Ferner ist es auch nicht ganz auszuschließen, dass ein Teil der Medikamentenflüssigkeit verlorengeht, bevor man die Ampulle in die für sie vorgesehene Aufnahme es Zuführungssystems einsetzen kann.

Die in der Offenlegungsschrift DE 10 2005 038 619 A1 beschriebene Erfindung versucht diese Nachteile zu überwinden, indem sie eine Inhalationstherapievorrichtung mit Ampulle umfassend eine Ampullenaufnahme vorschlägt, bei der die Ampulle im verschlossenen Zustand in die Aufnahme eingesetzt und erst beim Einsetzen mittels einer Öffnungseinrichtung in Form eines Dorns geöffnet wird.

Solange eine ausreichende Abdichtung der Verbindung von Ampulle und Öffnungseinrichtung gewährleistet ist, sind damit Verluste an Medikamentenflüssigkeit minimiert und auch eine Kontamination durch Keime aus der Umgebung wirksam verhindert.

Ein weiteres Problem hinsichtlich der Hygiene bleibt jedoch auch bei dieser Erfindung ungelöst. Um eine ausreichende Hygiene, d.h. Keimfreiheit, dauerhaft sicherzustellen sollten Vernebler in jedem Fall vor jeder Verwendung gereinigt und sterilisiert werden. Bei dem üblicherweise verwinkelten Aufbau üblicher Vernebler ist dies jedoch nur teilweise effektiv möglich, wodurch nach eine gewissen Verwendungsdauer, bzw. einer gewissen Anzahl Inhalationssitzungen, üblicherweise einige zehn Male entsprechend ca. eine bis höchstens zwei Wochen, die Keimfreiheit nicht mehr ausreichend gewährleistet ist.

Eine Lösung wäre, den Vernebler, oder zumindest die nicht ausreichend zuverlässig sterilisierbaren Teile, insbesondere die den Aerosolerzeuger umfassende Verneblereinheit, nach dieser maximalen sicheren Verwendungsdauer zu ersetzen. Dies ist jedoch problematisch, da im Stand der Technik bekannte Verneblereinheiten, auch und besonders die in den oben genannten Druckschriften offenbarten, aufgrund ihres komplexen Aufbaus recht teuer sind und somit für die Mehrzahl der Anwender eine Neuanschaffung nach der recht kurzen Benutzungsdauer von 1-2 Wochen nicht in Frage käme.

Die Patentschrift DE 10 2008 054 431 B3 zeigt eine Aerosoltherapievorrichtung mit einer Halterung für eine Medikamentenampulle, welche beim Einstecken von einem mit einer zuführungsseitig eines Aerosolgenerators gelegenen Kammer umschließenden Dorn aufgeschnitten wird, so dass Medikamentenflüssigkeit aus der Ampulle in die Kammer fließen und dort durch den Aerosolgenerator vernebelt werden kann. Aerosolgenerator und Aufschneidedorn sind gemeinsam einem Grundkörper zugeordnet, wohingegen die Ampullenhalterung in einem mit dem Grundkörper lösbar verbindbaren Deckel angeordnet ist.

Die Offenlegungsschrift DE 10 2005 038 619 A1 offenbart eine Inhalationstherapievorrichtung mit einer Verneblungseinrichtung, zu welcher Medikamentenflüssigkeit aus einer mittels eines Öffnungseinrichtung aufschneidbaren Ampulle zugeführt wird. Die Ampulle ist in einer Aufnahme in einem lösbar verbindbaren Deckel der Inhalationsvorrichtung gehalten.

Die veröffentlichte Anmeldeschrift EP 2 062 608 A2 beschreibt eine Einwegampulle zum Einsatz in einer Aerosolerzeugungsvorrichtung.

In der Veröffentlichungsschrift EP 2 987 520 A1 ist eine Flüssigkeitsreservoir für einen Vernebler und ein Vernebler mit einem solchen Reservoir offenbart, wobei das Reservoir im Inneren einen Kragen aufweist, durch den ein äußerer erster und ein innerer zweiter Bereich definiert sind, wodurch eine zum Aerosolerzeuger gelangende Flüssigkeitsmenge mehr oder weniger unabhängig von der Ausrichtung des Verneblers ist. In manchen Ausführungsformen hat das Reservoir die Form einer Ampulle, welche beim Einsetzen durch einen in einer Aufnahme gelegenen hohlen Dorn aufgeschnitten wird. Der hohle Dorn bildet im eingesetzten Zustand des Ampullen-Reservoirs den Kragen und umschließt somit den zweiten Bereich, der nach unten hin von dem Aerosolerzeuger begrenzt ist. Der Aerosolerzeuger ist zwischen einem an ein Mundstück anschließbaren vorderen Gehäuseteil und einem die Aufnahme und den Dorn umfassenden hinteren gehäuseteil eingeklemmt.

Die Patentschrift US 5,819,730 zeigt eine Vorrichtung zum verabreichen pharmazeutischer Substanzen, welche in flüssiger Form in Ampullen aufbewahrt werden. Die Ampullen werden durch Quetschen in der Vorrichtung geöffnet, woraufhin die durch die Quetschung unter Druck gesetzte Medikamentenflüssigkeit herausgedrückt wird.

Die veröffentlichte Patentanmeldung US 2003/0140921 A1 beschreibt einen Vernebler, in dem Ampullen zum Einsatz kommen.

Die Patentschrift US 6,145,703 zeigt einen Spray-Applikator für mehrfache Dosenabgaben von in einem versiegelten Behälter aufbewahrter Medikamentenflüssigkeit, welcher beim Einsetzen in eine Halterung von einer hohlen Nadel stirnseitig durchstochen wird.

Die internationale Veröffentlichungsschrift WO 96/28205 schlägt einen Vernebler mit einer Präzisionsdosiereinheit vor. Die Aufgabe, welche sich vorliegende Erfindung vor diesem Hintergrund gestellt hat, ist es, eine Verneblereinheit zu entwickeln, welche bei zumindest ebenbürtiger Hygiene und Dosiergenauigkeit eine benutzerfreundliche Verwendung erlaubt und simpel und kostengünstig herstellbar ist.

Gelöst wird diese Aufgabe durch eine Verneblereinheit mit Ampulle nach Anspruch 1 sowie einem Verfahren zu ihrer Herstellung gemäß Anspruch 12.

Eine wichtige Idee vorliegender Erfindung ist, die Ampulle mit einem Anschlussbereich auszustatten, durch den sie mithilfe der Anschlussmittel der Ampullenaufnahme direkt und unmittelbar an die Verneblereinheit anschließbar ist. Dies schließt nicht aus, dass Ampullen, welche über einen Anschlussbereich verfügen, welcher nicht unmittelbar mit den Anschlussmitteln der Ampullenaufnahme kompatibel ist, über einen zwischengeschalteten Adapter mit der erfindungsgemäßen Verneblereinheit verbunden werden können.

Der Anschlussbereich der Ampulle kann hierbei ein Außengewinde sein, welches mit einem entsprechend dimensionierten Innengewinde der Ampullenaufnahme kämmt, oder er umfasst ein oder mehrere Paar hervorstehende Knöpfe eines Bajonettverschlusses oder auch einen Ringwulst oder -nut eines Steckverschlusses.

Der Anschlussbereich ist hierbei üblicherweise in der Nähe einer Ausflussöffnung einer eingesetzten Ampulle bzw. in der Nähe einer zur Aufnahme einer solchen Ausflussöffnung vorgesehenen Stelle ausgebildet. Beispielsweise kann die Ampulle einen hohlzylinderförmiges Ende aufweisen, bei dem die Stirnseite zur Aufnahme einer Ausflussöffnung vorgesehen ist und an der Mantelfläche als Anschlussmittel ein Außengewinde eines Schraubverschlusses, ein Paar Knöpfe eines Bajonettverschlusses, ein Ringwulst oder eine Ringnut eines Steckverschlusses vorhanden sein.

Die Ausflussöffnung wird hierbei erst beim erfindungsgemäßen Anschließen der Ampulle mittels der Öffnungseinrichtung in das Material der Ampulle geschnitten oder gestanzt. Die Ampulle ist vor dem Einsetzen und Anschließen also noch vollständig geschlossen und versiegelt, wodurch Kontamination oder Flüssigkeitsverlust vermieden wird.

Die Form der Ampulle wie auch des Anschlussbereiches oder der Öffnung sind im Prinzip beliebig. Aus Gründen der Minimierung des Materialaufwandes bei gegebenen Flüssigkeitsvolumen bietet sich jedoch eine rotationssymmetrische Ampullenform, insbesondere Kugel- oder zumindest hohlzylindrische Form an. Um die bestmöglichen Versiegelung zu erreichen ist eine runde Öffnung empfehlenswert, da dies den abzudichtenden Umfang bei gegebener Öffnungsfläche minimiert.

Der mit der Öffnungseinrichtung in Kontakt kommende Bereich muss hierzu aus hinreichend weichem und schneidbaren Material bestehen, beispielsweise aus Kunststoff oder dünnem Metall. Der Rest der Ampulle, inklusive des Anschlussbereichs, kann entweder aus dem gleichen Material gefertigt sein, allerdings eventuell mit verschiedenen Wandstärken, wobei der Bereich in dem die Öffnung eingeschnitten werden soll, vorteilhafterweise dünnwandiger gestaltet wird, um das Anschließen der Ampulle zu erleichtern und ferner eine sauber definierte Öffnung zu garantieren. Dies ist für die Dichtigkeit der Verbindung zwischen Ampulle und Vernebler wichtig, insbesondere, wenn diese nur durch den Formschluss zwischen herausgeschnittener Öffnung und der Öffnungseinrichtung zustande kommt. Der Rest der Ampulle kann auch aus einem, oder mehreren anderen Materialien gefertigt sein. Es ist hier denkbar, ein festeres, stabileres Material wie Glas oder Metall vorzusehen. Die Ampulle kann beispielsweise als Glasfläschchen ausgebildet sein, welches an einem oberen Ende mit einem Siegel aus Metallfolie oder Kunststoff verschlossen ist.

In jedem Fall entfallen durch die erfindungsgemäße Gestaltung der Verneblereinheit und Ampullen zusätzliche bewegliche Teile und Mechaniken, welche bisher zum Einsetzen und Verbinden von Ampullen benötigt wurden. Die erfindungsgemäße Verneblereinheit ist dadurch zum einen einfacher und wesentlich kostengünstiger in der Herstellung, was den ,Amortisierungszeitraum` aus Sicht der Anwender verkürzt und zum anderen auch wesentlich weniger verwinkelt aufgebaut, so dass Reinigung und Sterilisierung vereinfacht sind. Somit ist gewährleistet das über einen Einsatzzeitraum von ein bis zwei Wochen, maximal einem Monat, keine oder nur eine sehr geringe Keimbelastung vorliegt.

Nach Ablauf dieses Zeitraums sollte die Verneblereinheit entsorgt und durch eine neue ersetzt werden. Aufgrund der günstigeren Herstellung wäre dies für einen Großteil der Anwender auch finanziell akzeptabel, was die Chance deutlich erhöht, dass dieser Empfehlung auch tatsächlich Folge geleistet wird.

Die Verwendung der erfindungsgemäßen Verneblereinheit sieht vor, eine bereitgestellte, mit Medikamentenflüssigkeit gefüllte Ampulle bestimmungsgemäß in die Ampullenaufnahme des Verneblers einzusetzen und durch ein Zusammenwirken der Anschlussmittel der Ampullenaufnahme mit dem Anschlussbereich der Ampulle an die Verneblereinheit anzuschließen, so dass die Ausflussöffnung der Ampulle, welche gemäß vorliegender Erfindung erst beim Einstecken/Anschließen durch die Öffnungseinrichtung geschaffen wurde mit der oder den Zulauföffnungen der Öffnungseinrichtung in Deckung zu bringen und flüssigkeitsdicht zu verbinden.

Abhängig von der Art der Anschlussmittel und des Anschlussbereiches wären seitens des Anwenders verschieden Bewegungsabläufe erforderlich. Ein Simpler Reibungsschluss zwischen Ampulle und Ampullenaufnahme ist hier die einfachste denkbare Lösung. Einstecken und Anschließen wären dann in einem Zug durch eine geradlinige Einsteckbewegung ermöglicht. Ist die Ampullenaufnahme rotationssymmetrisch, ist auch eine zusätzliche Drehung beim Einstecken ermöglicht, welche beispielsweise zur Unterstützung der Öffnungs- oder Aufschneidewirkung der Öffnungseinrichtung vorteilhaft sein kann.

Ein Schraubverschluss, bei dem ein Außengewinde der Ampulle mit einem Innengewinde der Ampullenaufnahme kämmend zusammenwirkt, verlangt, dass der Anwender die Ampulle in an sich bekannter Art mit dem Ende des Gewindes an das Innengewinde der Ampullenaufnahme ansetzt und mit Drehbewegungen in diese einschraubt, wobei die Ampulle an der dafür vorgesehenen Stelle, beispielsweise einem Bereich um die Drehachse auf einer vorderen Stirnseite, durch die Öffnungseinrichtung aufgeschnitten wird.

Bei einem Bajonettverschluss wäre zunächst ein geradliniges Einstecken gefolgt von einer Drehbewegung notwendig, wobei die Knöpfe, welche auf der Außenseite der Ampulle oder der Innenseite der Ampullenaufnahme ausgebildet sein können, zuvor entsprechend zu den Schlitzen ausgerichtet werden. Der Verlauf der Schlitze weist demgemäß einen axial gerichteten Abschnitt gefolgt von einem hauptsächlich tangentialen Abschnitt auf, wobei letztere durchaus einen nicht verschwindenden Winkel mit der Tangentialrichtung haben kann um beispielsweise den Aufschneidevorgang zu unterstützen.

Ein Steckverschluss mit zusammenwirkendem Ringwulst und Ringnut verlangt eine geradlinige Einsteckbewegung, erlaubt aber bei rotationssymmetrischer Ausführung zusätzlich eine die den Aufschneidevorgang durch die Öffnungseinrichtung unterstützende Drehbewegung.

"Ringwulst" oder "Ringnut" im hier verwendeten Sinne ist jede umlaufende Erhebung oder Depression, unabhängig davon ob sie einen kreisförmigen, ovalen, polygonalen oder anderen Querschnitt hat.

Die Herstellung der erfindungsgemäßen Verneblereinheit wird vereinfacht und beschleunigt, indem sie aus zwei wesentlichen Komponenten zusammengesetzt wird: einem Vorderteil, der den Aerosolerzeuger beherbergt und Anschlussmöglichkeiten für Kopplungskomponenten wie T-Stücke oder ein Mundstück umfasst, sowie einen Rückteil, welches die Ampullenaufnahme und Öffnungseinrichtung umfasst.

Zur Herstellung einer erfindungsgemäßen Verneblereinheit werden beide Teile durch Formen aus entsprechendem Material und gegebenenfalls Einbau der Komponenten in an sich bekannter Weise vorbereitet. Dann werden sie zusammengesetzt und entweder lösbar oder nicht lösbar verbunden. Ersteres kann durch Zusammenpressen oder Zusammenstecken (Form- und/oder Reibungsschluss), Verschrauben, letzteres durch Verkleben oder Verschweißen geschehen.

Vorteilhafte Weiterbildungen vorliegender Erfindung, welche Einzeln oder in Kombination realisierbar sind, sofern sie sich nicht offensichtlich gegenseitig ausschließen, sollen im Folgenden beschrieben werden.

Bevorzugt bilden die Anschlussmittel der Ampullenaufnahme und der damit zusammenwirkende Anschlussbereich der Ampulle einen Schraubverschluss, bei dem Ampullenaufnahme über ein Innen- und der Anschlussbereich über ein damit kämmendes Außengewinde verfügt. Alternativ bevorzugt wird die Ampulle mittels eine Bajonettverschlusses in der Ampullenaufnahme befestigt, wobei die Stifte oder Knöpfe als Teil der Ampullenaufnahme in deren Innenraum hineinstehend oder, besonders bevorzugt, als Teil des Anschlussbereichs der Ampulle ausgebildet sind. Die mit den Stiften bzw. Knöpfen zusammenwirkenden Schlitze sind dementsprechend im jeweils anderen Teil eingebracht, besonders bevorzugt in der Ampullenaufnahme.

Der Verlauf der Schlitze weißt hierbei bekannten Bajonettverschlüssen gemäß mindestens zwei Bereiche auf: einen ersten, im Wesentlichen axial und einen weiteren, im Wesentlichen tangential orientierten Bereich. Weiterhin bevorzugt schlägt vorliegende Erfindung vor, zwischen diesen beiden einen Bereich mit einem Winkel zur Tangentialrichtung von 5 bis 30 Grad, besonders bevorzugt 10 bis 20 Grad, vorzusehen. Dadurch wird erreicht, dass beim Einsetzen und Anschließen der Ampulle in diesem Zwischenbereich der Tangential- eine Axialbewegung überlagert. Diese kann vorteilhaft die Quetschung, d.h. heißt das teilweise Zusammenpressen, der Ampulle durch eine Quetscheinrichtung, im einfachsten Fall eine Verengung im Querschnitt der Ampullenaufnahme zusammenwirken. Bevorzugt erfolgt beim Einführen der Stifte im Anschlussbereich der Ampulle in den axialen Teil der korrespondierenden Schlitze der Ampullenaufnahme keine oder nur eine leichte Quetschung und die wesentliche Quetschung findet im Zuge der Drehung und axialen Verschiebung der Ampulle während des Durchlaufens des mittleren, gewinkelten Schlitzbereichs statt.

Eine weitere bevorzugte Alternative ist es, die Verbindung zwischen Ampulle und Verneblereinheit mittels eines Steckverschlusses herzustellen. Dieser umfasste eine umlaufende Ringnut und einen dazu korrespondierenden Ringwulst. Der Querschnitt des Wulstes ist bevorzugt größer als der der Ampullenaufnahme im oberen Bereich, wodurch eine Quetschung der Ampulle beim Einschieben und Anschließen garantiert ist.

Vorliegende Erfindung schlägt vor, dass in einer Ausführungsform die Öffnung in der Öffnungseinrichtung, durch welche die Medikamentenflüssigkeit aus der Ampulle in die Verneblereinheit einfließt, mit der unmittelbar vor dem Aerosolerzeuger gelegenen Kammer über einen Zuführungskanal kommuniziert. Dies schafft mehr Freiheit bei der baulichen Gestaltung der Verneblereinheit, da somit die Ausrichtung der Ampullenaufnahme und der darin vorhandenen Öffnungseinrichtung von der Ausrichtung des Aersolerzeugers unabhängig ist. Die fluide Kommunikation zwischen beiden ist in jedem Fall durch den Zuführungskanal sichergestellt.

Von vorliegender Erfindung bevorzugt steht die Zuführungsöffnung der Öffnungseinheit jedoch in direkter Kommunikation mit der Zuführungskammer, d.h. ohne Zwischenschaltung eines Zuführungskanals, da dies ein minimales zu füllendes Volumen ermöglicht und entsprechend eine kleiner dimensionierte Quetscheinrichtung oder Öffnungseinrichtung mit Verdrängerwirkung bereits ausreichend ist.

Die Öffnungseinrichtung der erfindungsgemäßen Verneblereinheit mit Ampulle ist bevorzugt ein Dorn, der über mindestens eine Zuführungsöffnung verfügt, welche mit der vor dem Aerosolerzeuger gelegenen Zuführungskammer in fluider Kommunikation steht. Besonders bevorzugt ist mehr als eine solche Zuführungsöffnung vorhanden. Damit kann ein gegebener bzw. gewünschter Öffnungsgesamtquerschnitt bei gleichzeitig kleinen Öffnungsradien realisiert werden.

Die Bezeichnung ,Dorn` soll hier implizieren, dass sein Oberseite geneigt ist, so dass er an einer Seite länger bzw. höher ist als auf einer anderen, beispielsweise der gegenüberliegenden. Die Oberseite kann hierbei eben gestaltet oder aber, besonders bevorzugt konkav gekrümmt ausgeführt sein. Dadurch wird die Spitze des Dorns prominenter und seine Aufschneidewirkung verbessert.

Weiterhin kann die Öffnungseinrichung, welche durch ihre Öffnung eine Richtung definiert, eine andere Ausrichtung aufweisen als der Aerosolerzeuger. So kann diese Richtung senkrecht zum Aerosolerzeuger stehen. Bevorzugt ist die Öffnungseinrichtung jedoch ein einem Winkel zwischen 30 und 60 zum Aerosolerzeuger orientiert. Dadurch können Kapillareffekte minimiert werden und es ist ein maximal schnelles und vollständiges Ausfließen der Medikamentenflüssigkeit aus der Ampulle garantiert. Dabei ist bei der Ausgestaltung der Ampulle darauf zu achten, dass bei einer inhalationsüblichen bzw. vorgeschriebenen Haltung des Verneblers, üblicherweise so, dass der Vernebler im Wesentlichen waagrecht ist, keine der Innenflächen der Ampulle eine zur horizontalen parallele Tangentialfläche aufweist.

Vorliegende Erfindung schlägt als weitere Ausgestaltungsmöglichkeit vor, eine Quetschung der Ampulle zu ergänzen oder zu ersetzen durch eine als Verdränger ausgeführte Öffnungseinrichtung. Hierbei würde die Öffnungseinrichtung gegenüber den bisher angesprochenen Ausführungen mit einem vergrößerten Volumen gestaltet. Dieses tritt beim Anschließen der Ampulle in den Innenraum hinein und verdrängt dort vorhandene Medikamentenflüssigkeit aus der Ampulle heraus und durch die Zuführungsöffnung oder -Öffnungen der Öffnungseinrichtung in die Zuführungskammer hinein. Eine solche Ausgestaltung hat den weiteren Vorteil, dass sich auch die Möglichkeit bietet, die Kontaktfläche zwischen einer Außenseite der Öffnungseinrichtung und der Ampulleninnenwand zu vergrößern, was der Dichtigkeit und Stabilität der Verbindung zu Gute kommt.

Die Verneblereinheit verfügt ferner auf der der Zuführungskammer gegenüberliegenden Seite des Aerosolerzeugers über eine Aerosolkammer, welche über eine Auslassöffnung verfügt. An diese Auslassöffnung für das Aerosol sind verschieden Kupplungsteile, wie beispielsweise Mundstücke zur direkten Inhalation oder auch T-Stücke eines Beatmungssystems anschließbar. Beispielsweise kann der die Aerosolkammer bildende Gehäuseteil als Hohlzylinder in Form eines Anschlussstutzens ausgeführt sein.

Vorliegende Erfindung sieht vor, die Verneblereinheit aus einem Vorder- und einem Rückteil zusammenzusetzen. Ersterer umfasst hierbei den Aerosolerzeuger, sowie die Aerosolkammer und Anschlussmöglichkeiten für Kupplungsstücke. Der Rückteil umfasst die Ampullenaufnahme mit Öffnungseinrichtung und die Zuführungskammer auf der Zuführungsseite des Aerosolerzeugers oder zumindest deren hinteren Teil. Die beiden Teile werden bei der Herstellung der erfindungsgemäßen Verneblereinheit zusammengebracht und miteinander verbunden. Im einfachsten Fall wird die Verbindung durch Reibungsschluss sichergestellt. Es kann jedoch auch eine ausgefeiltere lösbare Verbindungsmöglichkeit wie einen Nut und Wulst oder Nut und Feder umfassenden Steckverschluss, ein Bajonettverschluss oder ein Schraubverschluss vorgesehen sein. Besonders bevorzugt ist es allerdings, die beiden Teile nicht lösbar zu verbinden, indem sie nach dem Zusammenstecken verklebt oder Verschweißt werden. Dadurch ist die bestmögliche Dichtheit garantiert und auf zusätzliche Abdichtungsmittel, etwa Dichtringe an einer Kontaktfläche zwischen Vorder- und Rückteil, kann verzichtet werden. Das Gehäuse der erfindungsgemäßen Verneblereinheit ist bevorzugt aus Kunststoff, insbesondere einem thermoplastischen Kunststoff per Spritzguss hergestellt. Als Verschweißungsmethoden kommen daher Laserschweißen oder Ultraschallschweißen in Frage.

Zusätzlich zur reinen Verbindungs- und Anschlussfunktion kommt der Ampullenaufnahme auch eine Führungs- und Haltungsfunktion für die Ampulle zu. Um diese besser wahrzunehmen, schlägt vorliegende Erfindung vor, sie mit Ampullenführungen auszustatten, die komplementär zur Ampulle geformt sind.

Weiterhin ist in der Ampullenaufnahme erfindungsgemäß eine Quetscheinrichtung vorhanden, welche die Ampulle beim Einführen und Anschließen zu quetschen vermag, so dass im Inneren ein Überdruck aufgebaut wird, der ausreicht, um nach dem Öffnen, insbesondere Aufschneiden oder Aufstanzen, der Ampulle durch die Öffnungseinrichtung eine Menge Medikamentenflüssigkeit aus der Ampulle zu drücken, welche dem Volumen der Zuführungskammer und gegebenenfalls des Zuführungskanals entspricht. Die Quetscheinrichtung kann beispielsweise aus in den Innenraum der Ampullenaufnahme ragenden Noppen oder einem Wulst bestehen, oder auch in einem sich kontinuierlich verjüngenden Querschnitt der Ampullenaufnahme gegeben sein.

Die Abdichtung der Verbindung zwischen Ampulle und Verneblereinheit wird bevorzugt durch einen möglichst passgenauen Sitz der Ampulle gewährleistet, wobei die Ausflussöffnung der Ampulle die Öffnungseinrichtung dicht umschließt. Dass der Bereich in den die Öffnungseinrichtung die Ausflussöffnung in die Wand der Ampulle schneidet, aus flexiblem Material ist, trägt zu einem flüssigkeitsdichten Sitz bei.

Der passgenaue Sitz könnte hierbei vorteilhaft ergänzt oder die Anforderungen daran reduziert werden, wenn weitere Dichtmittel, etwa ein die Zuführungsöffnungen der Öffnungseinrichtung umschließenden Dichtring, welcher mit einer Stirnseite der Ampulle flächig abschließt.

Die Ampulle der erfindungsgemäßen Verneblereinheit mit Ampulle ist bevorzugt zumindest abschnittsweise aus einem flexiblen, leicht schneidbaren, jedoch ausreichend festen und stabilen Material gefertigt, insbesondere einem Kunststoff. Dies gilt insbesondere für den Bereich, in dem die Ausflussöffnung entstehen soll. Diese wird bevorzugt durch die Öffnungseinrichtung beim Einsetzen und Anschließen der erfindungsgemäßen Ampulle erzeugt. Alternativ kann die Ampulle im Bereich der zukünftigen Ausflussöffnung, insbesondere an ihrem unteren Ende, auch eine Abbruchlasche aufweisen, welche ein Anwender vor dem Einsetzen abbricht. Austreten von Medikamentenflüssigkeit würde dabei durch einen leichten Unterdruck in der Ampulle sichergestellt.

Weiterhin weist die Ampulle bevorzugt einen kreisförmigen Querschnitt auf, und ist besondere bevorzugt zumindest in dem in die Ampullenaufnahme eingeführten Teil rotationssymmetrisch geformt. Insbesondere ist die erfindungsgemäße Ampulle im Zustand vor dem Einsetzen grob hohlzylindrisch geformt. Am oberen Ende ist die Ampulle ferner bevorzugt zum leichteren Ergreifen und Eindrehen mit einer ergonomisch geformten, griffartigen Verdickung versehen.

Die offenbarte Verwendung sieht vor, dass eine mit Medikamentenflüssigkeit gefüllte Ampulle bereitgestellt und mit dem Anschlussbereich voran in die Ampullenaufnahme der erfindungsgemäßen Verneblereinheit eingesetzt und der jeweiligen Ausführungsform des Verschlusses gemäß angeschlossen wird. Beim Einsetz- und Anschlussvorgang wird gleichzeitig mittels der Öffnungseinrichtung eine Ausflussöffnung in der Wand der Ampulle, insbesondere einer Stirnseite, hergestellt, beispielsweise hineingeschnitten oder -gestanzt. Indem die Öffnungseinrichtung während des Schneidens oder Stanzens in die sich bildende Öffnung hineingepresst wird und diese vollständig ausfüllt, ist die Abdichtung gegen den Außenraum hin sichergestellt. Die Medikamentenflüssigkeit kann einzig durch die in der Öffnungseinrichtung vorhandenen Zuführungsöffnungen in die Zuführungskammer vor dem Aerosolerzeuger flie-ßen.

Weiterhin bevorzugt vorliegende Erfindung wenn dieses Ausfließen durch die Herstellung eines Überdrucks im Inneren der Ampulle unterstützt wird, und zwar durch Quetschung einer aus hinreichend flexiblem Material gefertigten Ampulle und/oder durch Verdrängung der Flüssigkeit aus dem Inneren der Ampulle mittels einer entsprechend voluminöser ausgestalteten, weiter in die Ampulle hineinragenden Öffnungseinrichtung. Letzteres bietet den Vorteil, auch bei Ampullen aus steifem Material, etwa aus Glas oder Metall, einsetzbar zu sein.

Das erfindungsgemäße Herstellungsverfahren umfasst die Bereitstellung eines den Aerosolerzeuger umfassenden Vorder- und eines die Ampullenaufnahme und Öffnungseinrichtung umfassenden Rückteils. Diese werden dann zusammengesetzt und lösbar oder nicht lösbar verbunden, wobei eine nicht lösbare Verbindung aus Gründen der Dichtheit und Hygiene bevorzugt ist. Eine lösbare Verbindung kann durch Reibungsschluss, Bajonett-, Steck- oder Schraubverbindung erreicht werden. Zur nicht lösbaren Verbindung schlägt vorliegende Erfindung Verkleben oder, bevorzugt, Verschweißen vor.

Weitere Eigenschaften, Merkmale und Vorteile vorliegender Erfindung ergeben sich aus den im folgenden Anhang der Figuren näher erläuterten Ausführungsbeispielen. Diese sollen die Erfindung nur illustrieren und in keiner Weise in ihrer Allgemeinheit einschränken.

Es zeigen:
- Figur 1:: Eine Ausführungsform einer erfindungsgemäßen Verneblereinheit mit einschraubbarer Ampulle.
- Figur 2:: Eine weitere Ausführungsform einer erfindungsgemäßen Verneblereinheit mit per Bajonettverschluss befestigter Ampulle.
- Figur 3:: Eine weitere Ausführungsform einer erfindungsgemäßen Verneblereinheit mit per Steckverschluss befestigter Ampulle.
- Figur 4:: Herstellung einer Ausführungsform der erfindungsgemäßen Verneblereinheit mit Ampulle aus Vorderteil und Rückteil.

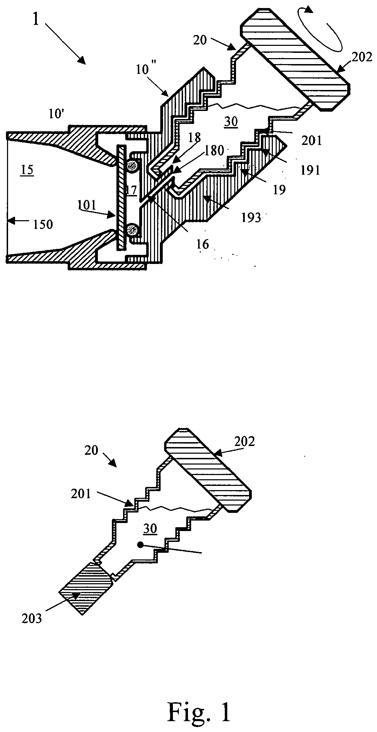

**Figur 1** zeigt einen Längsschnitt durch eine Ausführungsform der erfindungsgemäßen Verneblereinheit mit Ampulle. Gehäuse 10 besteht aus Vorderteil 10` und Rückteil 10". Der Vorderteil 10` beherbergt Aerosolerzeuger 101, auf dessen Zuführungsseite befindet sich Zuführungskammer 17, in welcher die Medikamentenflüssigkeit vor der Zerstäubung die Rückseite des Aerosolerzeugers benetzt. Ausgangsseitig ist die Aerosolkammer 15 mit Auslassöffnung 150, welche von einem im Wesentlichen als Hohlzylinder geformten Bereich des Gehäuseteils 10' umschlossen wird. Durch diese Formgebung ist dieser Teil zur Kopplung mit Kopplungsstücken wie einem Mundstück oder auch einem T-Stück einer Beatmungsanlage geeignet. Die rückwärtige Begrenzung der Kammer 17 ist durch eine Stirnfläche des Rückteils 10" gebildet. In dieser Begrenzung mündet die Zufühungsöffnung 180 der Öffnungseinrichtung 18, die wie die Ampullenaufnahme 19 zum Rückteil 10" gehört. Ampullenaufnahme 19 umfasst als Anschlussmittel 19 ein Innengewinde, welches mit einem Außengewinde 201 als Anschlussbereich einer eingesetzten Ampulle 20 kämmt und so die Ampulle fest in der Ampullenaufnahme hält. Öffnungseinrichtung 18, ein Dorn mit ebener Oberseite und einer konzentrischen Öffnung, wird umseitig vom Innenrand der Ausflussöffnung 200 von Ampulle 20 berührt, so dass ein fester und dichter Sitz gewährleistet ist.

Die Ampulle 20 mit Medikamentenflüssigkeit 30 ist im Wesentlichen rotationssymmetrisch und verfügt in dieser Ausführungsform über eine als Griff dienende Verdickung 202 am oberen und eine Abbruchlasche 203 am unteren Ende. Entfernt ein Anwender Abbruchlasch 203 entsteht Ausflussöffnung 200, welche von ihren Abmessungen den Außenabmessungen der Öffnungseinrichtung 18 entspricht.

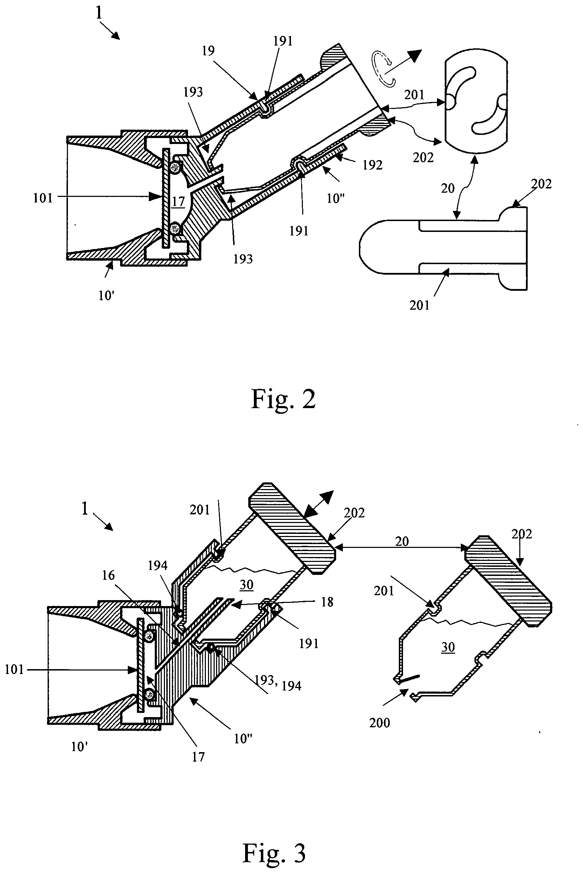

**Figur 2** zeigt einen Längsschnitt durch eine alternative Ausführungsform der erfindungsgemäßen Verneblereinheit mit Ampulle. Ampulle 20 wird mittels eines Bajonettverschlusses befestigt, bei dem die Stifte die Anschlussmittel 191 der Ampullenaufnahme 19 darstellen. Die Stifte greifen in Schlitze 201 in der Ampulle 20 ein, welche jeweils einen axialen ersten und einen tangentialen zweiten Abschnitt aufweisen, so dass die durch den Doppelpfeil angedeutete, entsprechende Bewegung zum Einsetzen und Anschließen der Ampulle 20 nötig ist. Ampullenaufnahme 19 ist über die Stifte 191 hinaus verlängert. Dieser Teil 192 dient der Führung und dem sicheren Halten von Ampulle 20. Das Gehäuse umfasst auch hier wiederum einen vorderen Teil 10` und einen rückwärtigen Teil 10". Öffnungseinrichtung 18 ist ein Dorn mit gekrümmter Oberseite, so dass die Spitze prominenter hervorsteht und leichter die Wandung der Stirnseite von Ampulle 20 einschneiden kann um Ausflussöffnung 200 zu schaffen. Quetscheinrichtung 193 presst oder quetscht den vorderen Teil der Ampulle zusammen, um im Inneren einen Überdruck zu schaffen, der einen dem Volumen von Zuführungskammer entsprechenden Teil der Flüssigkeit 30 aus dem Inneren von Ampulle 20 hinausdrückt.

In **Figur 3** ist noch eine weitere Ausführungsform der erfindungsgemäßen Verneblereinheit mit Ampulle im Längsschnitt dargestellt, bei welcher die Ampulle mittels eines Steckverschlusses gehalten wird. Ein Ringwulst 191 in der Ampullenaufnahme dient als Anschlussmittel, welches mit der Ringnut 201 im Anschlussbereich der Ampulle 20 zusammenwirkt. Ampulle 20 besitzt ein verdicktes ende, um eine unangenehme punktuelle Druckbelastung der Hand eines Anwenders beim Einstecken der Ampulle zu vermeiden und zudem beim Herausziehen eine bessere Angriffsmöglichkeit zu bieten. Öffnungseinrichtung 18 ist auch hier als Dorn mit gekrümmter Oberseite ausgebildet, ist jedoch gegenüber anderen Ausführungen wesentlich voluminöser. Dadurch kann sie als Verdränger dienen, welcher eine Menge Medikamentenflüssigkeit 30 aus dem Inneren von Ampulle 20 heraus- und über Ausflussöffnung 200, Zuführungsöffnung 180 und Zuführungskanal 16 in die Zuführungskammer 17 hinein verdrängt. Dies erlaubt den Einsatz von Ampullen aus wenig oder nicht-flexiblem Material wie Glas oder Metall, bei welchen eine zerstörungsfreie Quetschung nicht möglich ist. Dichtring 194 liegt die Öffnung 200 umschließend am unteren Ende der Ampulle 20 an und verhindert zusätzlich zum passgenauen Sitz auf Öffnungseinrichtung 18 ein Austreten von Medikamentenflüssigkeit.

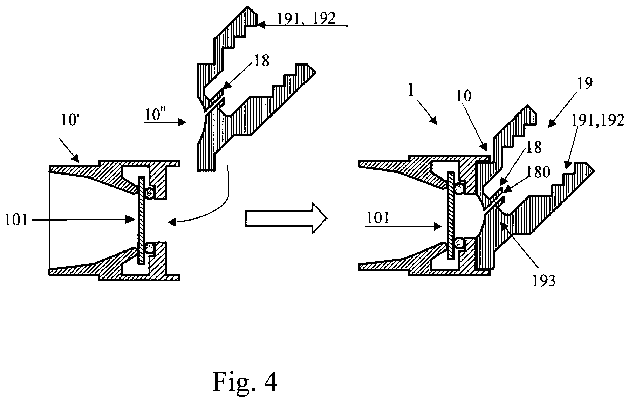

**Figur 4** zeigt in zwei Schritten eine Ausgestaltung der Herstellung einer erfindungsgemäßen Verneblereinheit aus einem Vorder- und einem Rückteil. Die separat gefertigten Teile 10', 10" werden zusammengesetzt und an der Kontaktstelle verschweißt um ein dicht schließendes, hygienisch einschließendes Gehäuse 10 zu bilden. Vorderteil 10' hält in seinem Inneren Aerosolerzeuger 101. Wie bei allen übrigen gezeigten Ausführungsformen ist der ausgangsseitige Abschnitt von Teilgehäuse 10' als ungefährer Hohlzylinder gefertigt und erlaubt den Anschluss von Kupplungsteilen wie Mund- oder T-Stücken. Der hintere Gehäuseteil 10" umfasst Ampullenaufnahme mit Anschlussmitteln 191, hier in Form eines Innengewindes eines Schraubverschlusses, welches zugleich als Ampullenführung 192 dient.

### Bezuaszeichenliste

- 1: Verneblereinheit
- 10: Gehäuse
- 10': Vorderteil
- 10": Rückteil
- 101: Aerosolerzeuger
- 15 16: Zuführungskanal
- 17: Zuführungskammer
- 18: Öffnungseinrichtung
- 180: Zuführungsöffnung
- 19: Ampullenaufnahme
- 191: Anschlussmittel
- 192: Ampullenführung
- 193: Quetscheinrichtung
- 194: Dichtring
- 20: Ampulle
- 200: Ausflussöffnung
- 201: Anschlussbereich
- 202: Griff
- 203: Lasche
- 30: Medikamentenflüssigkeit

## Patentansprüche

1. Verneblereinheit mit Ampulle, umfassend
- ein Gehäuse (10), welches einen Aerosolerzeuger (101) mit auf einer Seite gelegener Zuführungskammer (17) einschließt,
- eine Ampullenaufnahme (19) mit einer Quetscheinrichtung (193),
- eine Öffnungseinrichtung (18) mit mindestens einer Öffnung (180), welche mit der Zuführungskammer (17) in Kommunikation steht, und
- eine Ampulle (20) mit Flüssigkeit (30), welche über mindestens einer Öffnung (200) verfügt mit der sie in die Ampullenaufnahme eingesetzt oder einsetzbar ist,
wobei die Ampulle (20) einen Anschlussbereich (201) aufweist, der mit Anschlussmitteln (191) der Ampullenaufnahme (19) zusammenwirkt, um die Ampulle (20) fest in der Ampullenaufnahme (19) zu halten und die Öffnungen (200) und (180) dicht schließend zu verbinden, und
wobei das Gehäuse (10) aus einem den Aerosolerzeuger (101) umfassenden Vorderteil (10') und einem vom Vorderteil (10') separaten, die Ampullenaufnahme (19) umfassenden Rückteil (10") aufgebaut ist, und
wobei die Quetscheinrichtung (193) geeignet ist, die Ampulle (20) beim Einsetzen zu quetschen und dadurch einen Überdruck in der Ampulle (20) zu erzeugen,
wobei die Öffnungseinrichtung (18) im Rückteil (10") integriert ist, und die Quetscheinrichtung (193) weiterhin dazu Z ausgelegt ist, die Ampulle (20) derart beim Einsetzen zu quetschen und im eingesetzten Zustand gequetscht zu halten, dass im Inneren der Ampulle (20) ein Überdruck aufgebaut wird, der ausreicht, um nach dem Öffnen der Ampulle (20) durch die Öffnungseinrichtung (18) eine dem Volumen der Zuführungskammer (17) und gegebenenfalls eines Zuführungskanals entsprechende Menge an Medikamentenflüssigkeit aus der Ampulle (20) zu drücken.

2. Vernebelereinheit nach Anspruch 1, **dadurch gekennzeichnet, dass** Anschlussbereich (201) und Anschlussmittel (191)
- Außengewinde und Innengewinde eines Schraubverschlusses,
- Knöpfe und Schlitze eines Bajonettverschlusses, oder
- Ringwulst und Ringnut eines Steckverschlusses sind.

3. Verneblereinheit nach Anspruch 1 oder 2, **dadurch gekennzeichnet, dass** Öffnung (180) in Öffnungseinrichtung (18) mit der Zuführungskammer (17) über einen Zuführungskanal (16) kommuniziert.

4. Verneblereinheit nach einem der vorhergehenden Ansprüche, **dadurch gekennzeichnet, dass** die Öffnungseinrichtung (18)
- ein Dorn, insbesondere ein Dorn mit gekrümmter Oberseite, ist, und/oder
- über mehr als eine Öffnung (180) verfügt.

5. Verneblereinheit nach einem der vorhergehenden Ansprüche, **dadurch gekennzeichnet, dass** die Öffnungseinrichtung (18) eine andere Ausrichtung als der Aerosolerzeuger (101) aufweist, bevorzugt in einem Winkel zwischen 30 und 60 Grad, insbesondere in einem Winkel von 45 Grad zum Aerosolerzeuger orientiert ist.

6. Verneblereinheit nach Anspruch 5, **dadurch gekennzeichnet, dass** die Öffnungseinrichtung (18) von solcher Größe ist, dass im vollständig eingesetzten Zustand der Ampulle (20) ein in diese hineinragendes Teilvolumen der Öffnungseinrichtung, etwas größer als ein Volumen der Zuführungskammer (17) ist.

7. Verneblereinheit nach einem der vorhergehenden Ansprüche, **dadurch gekennzeichnet, dass** Vorderteil (10') und Rückteil (10")
- lösbar, insbesondere durch Schrauben oder Stecken, oder
- nicht lösbar, insbesondere durch Verkleben oder Verschweißen, etwa Laser- oder Ultraschallschweißen, verbunden sind.

8. Verneblereinheit nach einem der vorhergehenden Ansprüche, **dadurch gekennzeichnet, dass** die Quetscheinrichtung (193) aus in den Innenraum der Ampullenaufnahme (19) ragenden Noppen oder einem Wulst besteht oder ein sich kontinuierlich verjüngender Querschnitt der Ampullenaufnahme (19) ist.

9. Verneblereinheit nach einem der vorhergehenden Ansprüche, **dadurch gekennzeichnet, dass** die Abdichtung zwischen Ampullenöffnung (200) und Ampullenaufnahme (19) oder Öffnungseinrichtung (18) durch Dichtungsmittel, insbesondere einen oder mehrere Dichtringe, und/oder über passgenauen Sitz der Öffnung (200) auf Öffnungseinrichtung (18) gewährleistet ist.

10. Verneblereinheit nach einem der vorgehenden Ansprüche, **dadurch gekennzeichnet, dass** die Ampulle (20) an einem oberen Ende eine als Griff dienende Verdickung (202) aufweist.

11. Verneblereinheit nach einem der vorgehenden Ansprüche, **dadurch gekennzeichnet, dass** die Ampulle (20)
- im in den Vernebler eingesetzten Zustand bei einer vorgeschriebenen, optimalen Ausrichtung der Verneblereinheit nur Tangentialebenen an die Oberfläche ihres Innenvolumens aufweist, die zur Horizontalen einen Winkel von größer 0 Grad bilden, und/oder
- an einem unteren Ende eine Lasche zum Abbrechen aufweist, und/oder
- zumindest abschnittsweise aus flexiblem Material, insbesondere Kunststoff, gefertigt ist, und/oder
- einen kreisförmigen Querschnitt, und/oder
- eine rotationssymmetrische Form, und/oder
- eine im nicht eingesetzten Zustand grob hohlzylindrische Form aufweist.

12. Verfahren zur Herstellung einer Verneblereinheit gemäß einem der vorhergehenden Ansprüche, **dadurch gekennzeichnet, dass**
- ein Vorderteil (10') mit Aerosolerzeuger (101) und ein vom Vorderteil (10') separates Rückteil (10") mit Ampullenaufnahme (19) und Öffnungseinrichtung (18) bereitgestellt,
- zusammengesetzt, und
- lösbar oder nicht lösbar miteinander verbunden werden.

13. Verfahren nach dem vorhergehenden Anspruch, **dadurch gekennzeichnet, dass** die Verbindung von Vorderteil (10') und Rückteil (10") durch
- Reibungsschluss, und/oder
- durch Bajonett-, Steck- oder Schraubverschluss, und/oder
- durch Kleben und/oder Schweißen, insbesondere Laser- oder Ultraschallschweißen, erfolgt.

## Claims

1. A nebulizer unit with an ampoule, comprising
- a housing (10) which encases an aerosol generator (101) with a feeding chamber (17) lying on one side,
- an ampoule receiver (19) with a squeezing device (193),
- an opening device (18) with a least one opening (180) which is in communication with the feeding chamber (17), and
- an ampoule (20) with liquid (30), which possesses at least one opening (200) with which it is inserted or insertable into the ampoule receiver,
whereby the ampoule (20) has a connection region (201) that cooperates with connection means (191) of the ampoule receiver (19) to firmly retain the ampoule (20) in the ampoule receiver (19) and to sealingly connect the openings (200) and (180), and
whereby the housing (10) is made up of a front part (10`) that comprises the aerosol generator (101) and a rear part (10") that is separate from the front part (10`) and comprised the ampoule receiver (19), and whereby the squeezing device (193) is suitable for squeezing the ampoule (20) during its insertion, thereby creating an excess pressure in the ampoule (20),
whereby the opening device (18) is integrated into the rear part (10"), and
the squeezing device (193) is furthermore designed to squeeze the ampoule (20) during its insertion and hold it squeezed in the inserted state in such a way, that in an interior of the ampoule (20) an excess pressure is built which is sufficient to push, after the ampoule (20) has been opened by the opening device (18), an amount of medicament liquid corresponding to a volume of the feeding chamber and, if applicable, a feeding channel out of the ampoule (20).

2. The nebulizer according to claim 1, **characterized in that** the connection region (201) and the connection means (191) are
- an outer and an inner thread of a screw closure,
- pins and slots of a bayonet lock, or
- a ring bead and a ring groove nut of a plug-in closure.

3. The nebulizer according to claim 1 or 2, **characterized in that** the opening (180) in the opening device (18) communicates with the feeding chamber (17) via a feeding channel (16).

4. The nebulizer unit according to one of the proceeding claims, **characterized in that** the opening device (18)
- is a mandrel, in particular a mandrel with a curved top, and/or
- possesses more than one opening (180).

5. The nebulizer unit according to one of the proceeding claims, **characterized in that** the opening device (18) has a different orientation than the aerosol generator (101), preferably is oriented in an angle of between 30 and 60 degrees, in particular in an angle of 45 degrees relative to the aerosol generator.

6. The nebulizer unit of claim 5, **characterized in that** the opening device (18) is of such size that in the completely inserted state of the ampoule (20) a partial volume of the opening device protruding into the ampoule is slightly larger than a volume of the feeding chamber (17).

7. The nebulizer unit according to one of the proceeding claims, **characterized in that** the front part (10') and rear part (10") are connected
- releasably, in particular by screwing or plugging, or
- non-releasably, in particular by glueing or welding, like laser or ultrasound welding.

8. The nebulizer unit according to one of the proceeding claims, **characterized in that** the squeezing device (193) consists of nubs or a bead protruding into the interior of the ampoule receiver (19) or is a continuously tapering cross-section of the ampoule receiver (19).

9. The nebulizer unit according to one of the proceeding claims, **characterized in that** the seal between the ampoule opening (200) and the ampoule reveiver (19) or the opening device (18) is ensured by a sealing means, in particular one or several gaskets, and/or through a perfect fit of the opening (200) on the opening means (18).

10. The nebulizer unit according to one of the proceeding claims, **characterized in that** the ampoule (20) has at its upper end a bulge (202) serving as a grip.

11. The nebulizer unit according to one of the proceeding claims, **characterized in that** the ampoule (20)
- has, when inserted into the nebulizer and given the prescribed optimal orientation of the nebulizer unit, only tangential planes to the surface of its interior volume which form an angle of greater than 0 degrees with the horizontal, and/or
- has a break-off tab at its lower end, and/or
- is made at least partially from a flexible material, particularly a plastic, and/or
- has a circular cross-section, and/or
- a rotationally symmetrical shape, and/or
- a roughly hollow-cylindrical shape when not inserted.

12. A method for making a nebulizer unit according to one of the proceeding claims, **characterized in that**
- a front part (10`) with an aerosol generator (101) and a rear part (10") which is separate from the front part (10') and has an ampoule receiver (19) and an oppening device (18) are provided,
- assembled, and
- connected to each other in a releasable or non-releasable fashion.

13. The method according to the proceeding claim, **characterized in that** the connection of the front part (10`) and the rear part (10") is effected through
- a friction closure, and/or
- through a bayonet-, plug- or screw closure, and/or
- by glueing and/or welding, in particular laser or ultra sound welding.

## Revendications

1. Unité de nébulisation avec une ampoule, comprenant
- un boîtier (10) renfermant un générateur d'aérosol (101) avec une chambre d'alimentation (17) sur un côté,
- un réceptacle d'ampoule (19) avec un dispositif de pressage (193),
- un dispositif d'ouverture (18) avec au moins une ouverture (180) qui est en communication avec la chambre de d'alimentation (17), et
- une ampoule (20) avec du liquide (30) qui a au moins une ouverture (200) avec laquelle elle est ou peut être insérée dans le porte-ampoule,
dans lequel l'ampoule (20) présente une zone de connexion (201) qui coopère avec des moyens de connexion (191) du réceptacle d'ampoule (19) afin de maintenir fermement l'ampoule (20) dans le réceptacle d'ampoule (19) et de fermer les ouvertures (200 ) et (180) étroitement pour se connecter, et
dans lequel le boîtier (10) est construit à partir d'une partie avant (10') contenant le générateur d'aérosol (101) et d'une partie arrière (10"), qui est séparée de la partie avant (10') et contient le réceptacle d'ampoule (19 ), et
dans lequel le dispositif de pressage (193) est adapté pour presser l'ampoule (20) lorsqu'elle est insérée et générer ainsi une surpression dans l'ampoule (20),
dans lequel le dispositif d'ouverture (18) est intégré dans la partie arrière (10"), et
le dispositif de pressage (193) est également conçu pour presser l'ampoule (20) pendant l'insertion et pour la maintenir pressée dans l'état inséré de telle sorte qu'une surpression s'établisse à l'intérieur de l'ampoule (20) qui est suffisante pour presser, lors de l'ouverture de l'ampoule ( 20) par le dispositif d'ouverture (18), une quantité de liquide médicamenteux correspondant au volume de la chambre d'alimentation (17) et, le cas échéant, d'un canal d'alimentation hors de l'ampoule (20).

2. Unité de nébulisation selon la revendication 1, **caractérisée en ce que** la zone de connexion (201) et les moyens de connexion (191) sont
- un filetage extérieur et un filetage intérieur d'un bouchon à vis,
- des boutons et des fentes d'une fermeture à baïonnette, ou
- un boudin anneau et une rainure anneau d'une fermeture a fiche.

3. Unité de nébulisation selon la revendication 1 ou 2, **caractérisée en ce que** l'ouverture (180) du dispositif d'ouverture (18) communique avec la chambre d'alimentation (17) via un canal d'alimentation (16).

4. Unité de nébulisation selon l'une des revendications précédentes, **caractérisée en ce que** le dispositif d'ouverture (18)
- est un mandrin, en particulier un mandrin à sommet incurvé, et/ou
- a plus d'une ouverture (180).

5. Unité de nébulisation selon l'une des revendications précédentes, **caractérisé en ce que** le dispositif d'ouverture (18) a une orientation différente de celle du générateur d'aérosol (101), de préférence selon un angle compris entre 30 et 60 degrés, notamment selon un angle de 45 degrés, au générateur d'aérosol.

6. Unité de nébulisation selon la revendication 5, **caractérisé en ce que** le dispositif d'ouverture (18) est d'une taille telle que lorsque l'ampoule (20) est complètement insérée, un volume partiel du dispositif d'ouverture faisant saillie dans celle-ci est légèrement supérieur à un volume de la chambre d'alimentation (17).

7. Unité de nébulisation selon l'une des revendications précédentes, **caractérisée en ce que** la partie avant (10') et la partie arrière (10") sont connecté de manière
- détachable, notamment par vissage ou emboîtement, ou,
- indétachable, notamment par collage ou soudage, tel que soudage laser ou ultrasons.

8. Unité de nébulisation selon l'une des revendications précédentes, **caractérisée en ce que** le dispositif de pressage (193) consiste en des picots ou un bourrelet faisant saillie à l'intérieur du réceptacle d'ampoule (19) ou est une section transversale continûment effilée du réceptacle d'ampoule (19).

9. Unité de nébulisation selon l'une des revendications précédentes, **caractérisée en ce que** l'étanchéité entre l'ouverture d'ampoule (200) et le réceptacle d'ampoule (19) ou le dispositif d'ouverture (18) est assurée par des moyens d'étanchéité, en particulier une ou plusieurs bagues d'étanchéité, et/ou par un emboîtement précis de l'ouverture (200) sur le dispositif d'ouverture (18).

10. Unité de nébulisation selon l'une des revendications précédentes, **caractérisée en ce que** l'ampoule (20) présente une surépaisseur (202) servant de poignée à une extrémité supérieure.

11. Unité de nébulisation selon l'une des revendications précédentes, **caractérisée en ce que** l'ampoule (20)
- lorsqu'il est inséré dans le nébuliseur et avec un alignement optimal prescrit de l'unité de nébulisation, il ne présente que des plans tangents à la surface de son volume interne, qui forment un angle supérieur à 0 degré avec l'horizontale, et/ou
- a une languette pour casser à une extrémité inférieure, et/ou
- est réalisé, au moins par tronçons, en matériau souple, notamment en matière plastique, et/ou
- a une section circulaire, et/ou
- une forme à symétrie de rotation, et/ou
- une forme à peu près cylindrique creuse lorsqu'il n'est pas inséré.

12. Procédé de réalisation d'un unité de nébulisation selon l'une des revendications précédentes, **caractérisé en ce que**
- une partie avant (10') avec un générateur d'aérosol (101) et une partie arrière (10"), distincte de la partie avant (10'), avec une réceptacle d'ampoule (19) et un dispositif d'ouverture (18) sont fourni,
- composé, et
- reliés l'un à l'autre de manière détachable ou indétachable.

13. Procédé selon la revendication précédente, **caractérisé en ce que** la liaison de la partie avant (10') et de la partie arrière (10") par
- engagement par friction, et/ou
- par connexion baïonnette, fiche ou vis, et/ou
- par collage et/ou soudage, notamment soudage laser ou ultrasons.
